# EUROPEAN PATENT APPLICATION

(11) **EP 4 706 657 A1**
(43) Date of publication of application: **11.03.2026**
(21) Application number: 24797475.1
(22) Date of filing: 26.04.2024
(51) Int. Cl.: A61K 31/519, A61P 11/00

(54) **COMBINATION THERAPY OF PEXIDARTINIB AND NINTEDANIB FOR PREVENTION OR TREATMENT OF PULMONARY FIBROSIS**

(30) Priority: 28.04.2023 KR 20230056517
(71) Applicant: Korea Institute of Radiological & Medical Sciences, Seoul 01812 (KR)
(72) Inventor: LEE, Yoon-Jin, Seoul 07997 (KR); LEE, Hae-June, Seoul 04978 (KR); KIM, Jihee, Seoul 03763 (KR); NAM, Jae Kyung, Seoul 01658 (KR)
(74) Representative: Mewburn Ellis LLP
(86) International application number: PCT/KR2024/005691
(87) International publication number: WO 2024/225821

(57) **Abstract**

The present invention relates to a combination therapy of pexidartinib and nintedanib for preventing or treating pulmonary fibrosis.

## Description

### [Technical Field]

The present invention relates to a combination therapy of pexidartinib and nintedanib for preventing or treating pulmonary fibrosis.

### [Background Art]

Pulmonary fibrosis refers to a condition in which fibrous connective tissue proliferates in the lungs, thereby destroying the normal lung structure and resulting in the hardening and deterioration of lung tissue.

In particular, idiopathic pulmonary fibrosis (IPF) is a disease in which chronic inflammatory cells infiltrate the alveolar walls, causing various changes that harden the lungs, induce severe structural alterations in lung tissue, and gradually deteriorate pulmonary function. To date, no effective therapeutic method exists.

In addition, radiotherapy is often used to treat patients with non-small cell lung cancer (NSCLC) who are not eligible for surgical resection, but it often causes radiation-induced pulmonary fibrosis (RIPF).

To date, Ofev (Boehringer Ingelheim) containing nintedanib as an active ingredient is known to attenuate the decline in lung function to some extent. However, there has been a growing need to develop more effective therapeutic agents.

### [Disclosure]

### [Technical Problem]

As a result of continuous efforts to develop a more effective drug for the prevention or treatment of pulmonary fibrosis, the present inventors have confirmed the preventive or therapeutic effect of a combination therapy of pexidartinib and nintedanib on pulmonary fibrosis, thereby completing the present invention.

### [Technical Solution]

One object of the present invention is to provide a therapy for preventing, improving, or treating pulmonary fibrosis, comprising co-administration of pexidartinib or a pharmaceutically acceptable salt or solvate thereof, and nintedanib or a pharmaceutically acceptable salt or solvate thereof.

Another object of the present invention is to provide a pharmaceutical composition for preventing or treating pulmonary fibrosis, containing pexidartinib or a pharmaceutically acceptable salt or solvate thereof, wherein the pharmaceutical composition is administered in combination with nintedanib, or a pharmaceutically acceptable salt or solvate thereof.

Still another object of the present invention is to provide a pharmaceutical composition for preventing or treating pulmonary fibrosis, containing nintedanib or a pharmaceutically acceptable salt or solvate thereof, wherein the pharmaceutical composition is administered in combination with pexidartinib, or a pharmaceutically acceptable salt or solvate thereof.

Still another object of the present invention is to provide a combination of pexidartinib or a pharmaceutically acceptable salt or solvate thereof, and nintedanib or a pharmaceutically acceptable salt or solvate thereof.

Still another object of the present invention is to provide a pharmaceutical composition for preventing, improving, or treating pulmonary fibrosis, comprising the combination.

Still another object of the present invention is to provide a pharmaceutical kit for preventing, improving, or treating pulmonary fibrosis, comprising the combination.

Still another object of the present invention is to provide a method for preventing, improving, or treating pulmonary fibrosis, comprising administering and/or using the combination, the pharmaceutical composition, or the pharmaceutical kit to a subject in need thereof.

Still another object of the present invention is to provide a method for preventing, improving, or treating pulmonary fibrosis, comprising co-administering and/or co-using to a subject in need thereof a composition comprising a pharmaceutically effective amount of pexidartinib or a pharmaceutically acceptable salt or solvate thereof, and a composition comprising nintedanib or a pharmaceutically acceptable salt or solvate thereof.

Still another object of the present invention is to provide use of the combination, pharmaceutical composition, or pharmaceutical kit for preventing, improving, or treating pulmonary fibrosis and/or for preparing a drug for preventing, improving, or treating pulmonary fibrosis.

### [Advantageous Effects]

The co-administration therapy of the present invention exhibits an enhanced effect compared to single administration therapies, and thus can be usefully employed in the prevention or treatment of pulmonary fibrosis.

### [Brief Description of the Drawings]

FIG. 1 shows the effect of inhibiting pulmonary fibrosis, confirmed by Micro-CT imaging, after administration of nintedanib, pexidartinib (IM-1), or a combination of nintedanib and pexidartinib (Nin+IM-1) to mice in which pulmonary fibrosis was induced by bleomycin.
FIG. 2 shows a graph illustrating the degree of inflammation and fibrosis, confirmed by H&E and Masson's Trichrome staining, after administration of nintedanib, pexidartinib, or a combination of nintedanib and pexidartinib to mice in which pulmonary fibrosis was induced by bleomycin.
FIG. 3 shows the results of confirming survival rates by treatment group after administration of nintedanib, pexidartinib, or a combination of nintedanib and pexidartinib to mice in which pulmonary fibrosis was induced by bleomycin.
FIG. 4 shows the effect of inhibiting radiation-induced pulmonary fibrosis (RIPF), confirmed by Micro-CT imaging, after administration of nintedanib, pexidartinib, or a combination of nintedanib and pexidartinib to mice in which pulmonary fibrosis was induced by radiation exposure.
FIG. 5 shows a graph illustrating the degree of inflammation and fibrosis, confirmed by H&E and Masson's Trichrome staining, after administration of nintedanib (Nintedanib), pexidartinib (Pexidartinib), or a combination of nintedanib and pexidartinib to mice in which pulmonary fibrosis was induced by radiation exposure.

### [Detailed Description of Preferred Embodiments]

The present disclosure will be described in detail as follows. Meanwhile, each description and embodiment described herein may be applied to other descriptions and embodiments, respectively. That is, all combinations of various elements described herein fall within the scope of the present invention. Further, the scope of the present invention is not limited by the specific description described below.

Furthermore, a person of ordinary skill in the art may recognize or identify numerous equivalents to the specific embodiments of the present invention described herein by using routine experimentation. Moreover, such equivalents are intended to be included within the present invention.

Additionally, a number of papers and patent documents have been cited throughout the present specification. The content of the cited papers and patent documents is incorporated herein by reference in their entirety to describe the level of the technical field to which the present invention belongs and the contents of the present invention more clearly.

One aspect of the present invention provides a therapy for preventing, improving, or treating pulmonary fibrosis, comprising co-administration of pexidartinib or a pharmaceutically acceptable salt or solvate thereof, and nintedanib or a pharmaceutically acceptable salt or solvate thereof.

Another aspect of the present invention provides a pharmaceutical composition for preventing or treating pulmonary fibrosis, containing pexidartinib or a pharmaceutically acceptable salt or solvate thereof, wherein the pharmaceutical composition is administered in combination with nintedanib or a pharmaceutically acceptable salt or solvate thereof.

Another aspect of the present invention provides a pharmaceutical composition for preventing or treating pulmonary fibrosis, containing nintedanib or a pharmaceutically acceptable salt or solvate thereof, wherein the pharmaceutical composition is administered in combination with pexidartinib or a pharmaceutically acceptable salt or solvate thereof.

The pexidartinib of the present invention, also referred to as PLX3397 or Turalio (trade name), is a commercially available compound having the structure represented by following Formula 1.

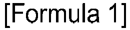

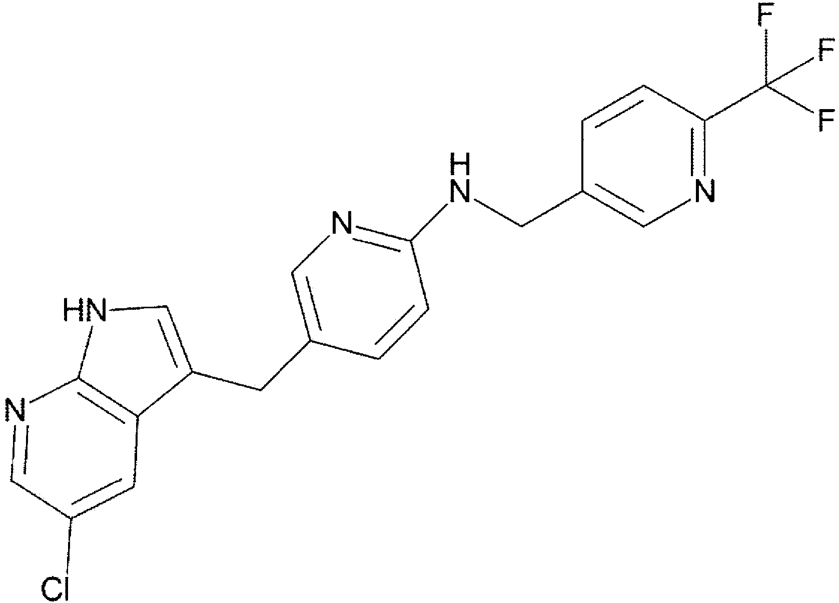

The IUPAC name of pexidartinib is 5-((5-chloro-1H-pyrrolo[2,3-b]pyridin-3-yl)methyl)-N-((6-(trifluoromethyl)pyridin-3-yl)methyl)pyridin-2-amine. Pexidartinib is a molecular targeted agent that specifically inhibits CSF-1R, KIT, and FLT3, and is known to exhibit effects such as inhibition of cancer cell proliferation and suppression of metastasis. In the present invention, pexidartinib may be referred to as "IM-1".

The nintedanib of the present invention, also referred to as BIBF1120, or Vargatef or Ofev (trade name), is a commercially available compound having the structure represented by following Formula 2.

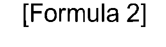

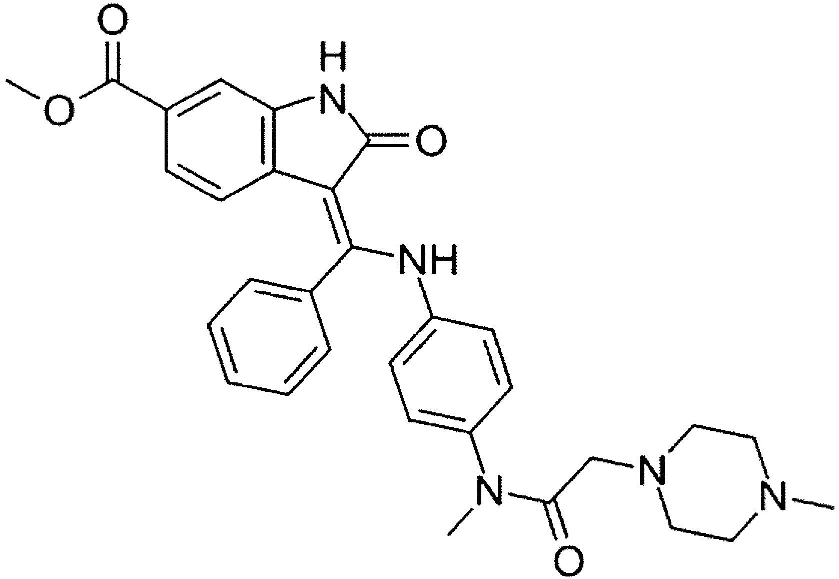

The IUPAC name of nintedanib is methyl (3Z)-3-{[(4-{methyl[(4-methylpiperazin-1-yl)acetyl]amino}phenyl)amino](phenyl)methylidene}-2-oxo-2,3-dihydro-1H-indole-6-carboxylate.

Meanwhile, the compounds of the present invention encompass the compounds with a specific chemical structure as well as their clathrates, hydrates, solvates, or polymorphs. Additionally, the compound of the present invention encompasses pharmaceutically acceptable salts thereof, even when the pharmaceutically acceptable salts are not explicitly mentioned. In one embodiment, the compound of the present invention may exist as a stereoisomerically pure compound (for example, substantially free of other stereoisomers (for example, at least 85% ee, at least 90% ee, at least 95% ee, at least 97% ee, or at least 99% ee)), but is not limited thereto.

The term "hydrate" refers to a compound of the present invention that includes a stoichiometric or non-stoichiometric amount of water bound by non-covalent intermolecular forces, or a pharmaceutically acceptable salt thereof.

The term "clathrate" refers to a compound of the present invention in the form of a crystal lattice that includes spaces (for example, channels) in which guest molecules (for example, solvent or water) are trapped, or a salt thereof.

The term "pharmaceutically acceptable" means that it is suitable for use as a pharmaceutical preparation, and may be used, within the scope of sound medical judgment, to refer to a compound, substance, composition, and/or form of administration that is suitable for contact with human or animal tissues without undue toxicity, irritation, allergic reaction, or other problems or complications and that has a reasonable benefit-to-risk ratio.

The term "pharmaceutically acceptable salt" in the present invention refers to a derivative of a known compound in which a parent compound is modified by forming an acid or base salt thereof. Examples of the pharmaceutically acceptable salt include mineral or organic acid salts of basic residues such as amines, and alkali or organic salts of acidic residues such as carboxylic acids, but are not limited thereto. Examples of the pharmaceutically acceptable salt of the present invention include commonly used salts in the pharmaceutical field, such as hydrochloride, hydrobromide, hydroiodide, hydrofluoride, sulfate, sulfonate, citrate, camphorate, maleate, acetate, lactate, nicotinate, nitrate, succinate, phosphate, malonate, malate, salicylate, phenylacetate, stearate, formate, fumarate, urea, sodium, potassium, calcium, magnesium, zinc, lithium, cinnamate, methylamino, methanesulfonate, picrate, p-toluenesulfonate, naphthalenesulfonate, tartrate, triethylamino, dimethylamino, and tris(hydroxymethyl)aminomethane salts, but are not limited thereto.

The pharmaceutically acceptable salt of the compound of the present invention may be synthesized from a parent compound comprising a basic or acidic moiety by using a conventional chemical method. Generally, such a salt may be prepared by reacting the compound in free acid or base form with a sufficient amount of a suitable base or acid in water or in an organic diluent such as ether, ethyl acetate, ethanol, isopropanol, or acetonitrile, or a mixture thereof.

The term "solvate" or "pharmaceutically acceptable solvate" refers to a solvate formed from the association of one or more solvent molecules with the compound. The term "solvate" includes hydrates (for example, hemihydrates, monohydrates, dihydrates, trihydrates, or tetrahydrates).

The pharmaceutical composition according to the present invention may be prepared by a conventional method used in the pharmaceutical field. The pharmaceutical composition may be formulated with a suitable pharmaceutically acceptable carrier depending on the dosage form, and may be prepared by further comprising an excipient, diluent, dispersant, emulsifier, buffer, stabilizer, binder, disintegrant, solvent, or the like, if necessary. The suitable carrier or the like does not impair the activity or properties of the compound according to the present invention, and may be selected according to the form of administration and dosage form.

The pharmaceutical composition of the present invention may further comprise a suitable carrier, excipient, or diluent that is commonly used in the preparation of a pharmaceutical composition. The composition comprising a pharmaceutically acceptable carrier may be in various oral or parenteral dosage forms. When formulated, the composition may be prepared using a commonly used diluent or excipient such as a filler, extender, binder, wetting agent, disintegrant, or surfactant. Solid preparations for oral administration may include tablets, pills, powders, granules, capsules, *etc.,* and such solid preparations may be formulated by mixing, into the composition, at least one or more excipients, such as starch, calcium carbonate, sucrose, lactose, and gelatin. Further, in addition to simple excipients, lubricants such as magnesium stearate and talc may be used. Examples of liquid preparations for oral administration may include suspensions, liquids for internal use, emulsions, syrups, *etc.,* and in addition to commonly used simple diluents including water and liquid paraffin, various excipients such as wetting agents, sweeteners, flavoring agents, and preservatives may be included. Preparations for parenteral administration may include sterilized aqueous solutions, non-aqueous solvents, suspensions, emulsions, freeze-dried preparations, and suppositories. As non-aqueous solvents and suspending agents, propylene glycol, polyethylene glycol, vegetable oils such as olive oil, injectable esters such as ethyl oleate, *etc.* may be used. As bases for suppositories, Witepsol, Macrogol, Tween 61, cocoa butter, lauric acid, glycerogelatin, *etc.* may be used.

In addition, the pharmaceutical composition of the present invention may, without being limited thereto, be in a dosage form selected from the group consisting of tablets, pills, powders, granules, capsules, suspensions, liquids for internal use, emulsions, syrups, sterile aqueous solutions, non-aqueous solvents, suspensions, emulsions, lyophilized preparations, and suppositories.

Each component of the pharmaceutical composition according to the present invention may be included in the pharmaceutical composition in a pharmaceutically effective amount.

The term "pharmaceutically effective amount" refers to an amount that is sufficient to inhibit or relieve increased vascular permeability with a reasonable benefit-risk ratio applicable to medical use. The effective dose level may be determined based on factors including the subject type, severity, age, sex, drug activity, sensitivity to drugs, administration time, route of administration and elimination rate, treatment duration, and combination or concurrent use of drugs, as well as other well-known factors in the medical field.

The effective dose level of the pharmaceutical composition may be determined based on factors including the target use, age, sex, weight, and health conditions of the patient, type and severity of the disease, drug activity, sensitivity to drugs, administration method, administration time, route of administration and elimination rate, treatment duration, and combination or concurrent use of drugs, as well as other well-known factors in the medical field. For example, although not consistent, it may generally be administered once or several times daily in an amount of 0.001 mg/kg to 100 mg/kg, for example, 0.01 mg/kg to 10 mg/kg. However, the administered dose does not limit the scope of the present invention by any means.

The pharmaceutical composition may be appropriately administered to a subject based on the conventional method, route of administration, and administered dose used in the art, depending on the purpose or need. Examples of the route of administration include oral, parenteral, subcutaneous, intraperitoneal, intrapulmonary, and intranasal administration. Parenteral injection comprises intramuscular, intravenous, intraarterial, intraperitoneal, or subcutaneous administration. Additionally, suitable dose and administration frequency may be selected according to a known method in the art. The actual amount and administration frequency of the pharmaceutical composition of the present invention to be administered may be appropriately determined based on various factors such as the type of symptom to be treated, route of administration, sex, health conditions, diet, age, and body weight of the subject, and severity of the disease.

The term "administration" refers to the introduction of the pharmaceutical composition of the present invention to a subject using a suitable method, and the route of administration includes various oral and parenteral routes, as long as it allows the composition to reach the target tissue.

The pharmaceutical composition may be administered to any animal in which pulmonary fibrosis may occur, and the animal may include, for example, livestock such as cattle, pigs, horses, and dogs, in addition to humans and primates. In certain embodiments, the animal may be an animal excluding humans.

The pharmaceutical composition may be administered through a suitable route of administration for the dosage form, and may be administered using various oral or parenteral routes, as long as it allows the composition to reach the target tissue. The administration method may, without being limited thereto, be administered using a conventional method including, for example, oral, rectal or intravenous, intramuscular, transdermal administration, inhalation, intrauterine epidural, or intracerebroventricular injection.

As used herein, the term "combination administration", "co-administration", or "administration in combination" not only refers to the simultaneous administration, but may also refer to a form of administration that allows pexidartinib or a pharmaceutically acceptable salt or solvate thereof, and nintedanib or a pharmaceutically acceptable salt or solvate thereof to act together in a subject such that each substance can perform its inherent function at an equivalent or higher level. Accordingly, as used herein, when the term "combination" is used, it refers to simultaneous, separate, sequential, or reverse administration, and is to be construed as being unrestricted in order. When the administration is sequential, reverse, or separate, the order of administration is not particularly limited, but the interval between the administration of the second ingredient should not lead to the loss of the beneficial effect of the combination administration.

In the present invention, (i) pexidartinib or a pharmaceutically acceptable salt or solvate thereof, and (ii) the composition comprising nintedanib or a pharmaceutically acceptable salt or solvate thereof may be administered in a form as described below, but are not limited thereto:
a) administration of a mixture in which (i) pexidartinib, or a pharmaceutically acceptable salt or solvate thereof, and (ii) nintedanib or a pharmaceutically acceptable salt or solvate thereof are mixed; or
b) administration of (i) pexidartinib, or a pharmaceutically acceptable salt or solvate thereof, and (ii) nintedanib or a pharmaceutically acceptable salt or solvate thereof in separate forms, but is not limited thereto.

When (i) pexidartinib, or a pharmaceutically acceptable salt or solvate thereof, and (ii) nintedanib or a pharmaceutically acceptable salt or solvate thereof are administered in separate forms, the (i) and (ii) may be formulated into separate formulations and administered simultaneously, separately, sequentially, or in reverse order.

The therapeutically effective dose of each active ingredient used in the combination administration may vary depending on the particular compound or pharmaceutical composition used, the mode of administration, the symptom to be treated, the severity of the symptom, and the species, body weight, sex, dietary conditions, and age of the warm-blooded animal. Accordingly, the administration regimen using the compounds of the present invention is selected depending on various factors, including the route of administration and the renal and hepatic functions of the patient. A surgeon, clinician, or veterinarian skilled in the art can readily determine and prescribe an effective amount of a drug required to prevent, counteract, or arrest the progression of symptoms. The optimal precision in achieving a drug concentration within the range that provides efficacy without toxicity requires a regimen based on the pharmacokinetics of drug availability at the targeted site. It includes taking into consideration the distribution, equilibrium, and elimination of the drug. Accordingly, the dosage regimen, that is, the administration level and frequency of any individual component of the present invention, may be adjusted to provide an optimal therapeutic response.

Another aspect of the present invention provides a combination comprising pexidartinib or a pharmaceutically acceptable salt or solvate thereof, and nintedanib or a pharmaceutically acceptable salt thereof.

As used herein, the term "combination" refers to the use for combination administration of pexidartinib or a pharmaceutically acceptable salt or solvate thereof, and nintedanib or a pharmaceutically acceptable salt or solvate thereof, and may be understood as having the same meaning as "combination use". This also encompasses, but is not limited to, a pharmaceutical composition and a pharmaceutical kit characterized by the combination of pexidartinib or a pharmaceutically acceptable salt or solvate thereof, and nintedanib or a pharmaceutically acceptable salt or solvate thereof.

As used herein, the term "kit" may comprise the combination or composition according to the present invention for the combination administration of pexidartinib or a pharmaceutically acceptable salt or solvate thereof, and nintedanib and a pharmaceutically acceptable salt or solvent thereof. Specifically, the kit of the present invention may comprise pexidartinib or a pharmaceutically acceptable salt or solvate thereof, and nintedanib or a pharmaceutically acceptable salt or solvate thereof as a single formulation, or comprise pexidartinib or a pharmaceutically acceptable salt or solvate thereof, and nintedanib or a pharmaceutically acceptable salt or solvate thereof as separate formulations. The kit may further comprise a substance necessary for the combination administration of the two substances. However, the kit is not limited thereto.

The term "pulmonary fibrosis" refers to a respiratory disease in which lung tissue becomes hardened, causing severe respiratory dysfunction. Pulmonary fibrosis may occur due to various causes such as radiation, tuberculosis, syphilis, pneumoconiosis, or viral infections, and may also occur for unknown reasons. All such pulmonary fibrosis falls within the scope of pulmonary fibrosis of the present invention.

In one embodiment of the present invention, the pulmonary fibrosis may be selected from pulmonary fibrosis caused by radiation exposure and idiopathic pulmonary fibrosis.

The term "idiopathic pulmonary fibrosis" (IPF) of the present invention refers to a disease of unknown cause among interstitial lung diseases, wherein repeated inflammation resulting from alveolar injury induces fibrosis and leads to respiratory failure in patients.

In one embodiment of the present invention, pulmonary fibrosis may be a side effect of radiotherapy in which normal tissues are also exposed to radiotherapy for cancer treatment, or a side effect of chemotherapy used for cancer treatment.

Examples of radiotherapy or chemotherapy for cancer treatment that may induce pulmonary fibrosis may include the treatment of breast cancer, lung cancer, bone cancer, pancreatic cancer, skin cancer, oral cancer, oropharyngeal cancer, uterine cancer, ovarian cancer, rectal cancer, stomach cancer, endometrial carcinoma, cervical carcinoma, vaginal carcinoma, small intestine cancer, thyroid cancer, parathyroid cancer, prostate cancer, chronic or acute leukemia, lymphocytic lymphoma, bladder cancer, kidney cancer, liver cancer, colon cancer, or brain tumors, but are not limited thereto.

In one embodiment, the pharmaceutical composition of the present invention may be administered before or after radiation exposure.

The term "treatment" refers to any activity that improves or beneficially changes a symptom of fibrosis by administering the pharmaceutical composition, and the term "prevention" refers to any activity that inhibits or delays the onset of fibrosis by administering the pharmaceutical composition.

As used herein, the term "improvement" refers to any activity that at least reduces a parameter associated with pulmonary fibrosis, for example, the severity of a symptom, through the administration of the composition of the present invention.

Still another aspect of the present invention provides a method for preventing, improving, or treating pulmonary fibrosis, comprising administering and/or using a combination, pharmaceutical composition, or pharmaceutical kit comprising pexidartinib or a pharmaceutically acceptable salt or solvate thereof, and nintedanib or a pharmaceutically acceptable salt or solvate thereof to a subject in need thereof.

Still another aspect of the present invention provides a method for preventing, improving, or treating pulmonary fibrosis, comprising co-administering and/or co-using to a subject in need thereof a composition comprising a pharmaceutically effective amount of pexidartinib or a pharmaceutically acceptable salt or solvate thereof, and a composition comprising nintedanib or a pharmaceutically acceptable salt or solvate thereof.

Still another aspect of the present invention provides use of a composition comprising a pharmaceutically effective amount of pexidartinib or a pharmaceutically acceptable salt or solvate thereof, and a pharmaceutical composition or pharmaceutical kit comprising nintedanib or a pharmaceutically acceptable salt or solvate thereof, for preventing, improving, or treating pulmonary fibrosis or for preparing a drug for preventing, improving, or treating pulmonary fibrosis.

The composition comprising pexidartinib or a pharmaceutically acceptable salt or solvate thereof, nintedanib or a pharmaceutically acceptable salt or solvate thereof, pulmonary fibrosis, prevention, and treatment are as described in other aspects above.

### [Mode for Carrying Out the Invention]

Hereinafter, the present invention will be described in more detail by way of Examples and Experimental Examples. However, these Examples and Experimental Examples are given for illustrative purposes only, and the scope of the present invention is not intended to be limited by these Examples and Experimental Examples.

### Example 1. Confirmation of inhibitory effect on pulmonary fibrosis induced by bleomycin

Bleomycin sulfate was administered at a dose of 1.5 U/kg to six-week-old male C57BL/6 mice, and the mice were observed for pulmonary fibrosis (10 mice per group). Each drug was administered starting 14 days after the administration of bleomycin. The control drugs, nintedanib and pexidartinib (IM-1), were both administered at a dose of 60 mg/kg, and the co-administration group was orally administered nintedanib (60 mg/kg) with IM-1 (60 mg/kg) once daily for two weeks (a total of 13 administrations).

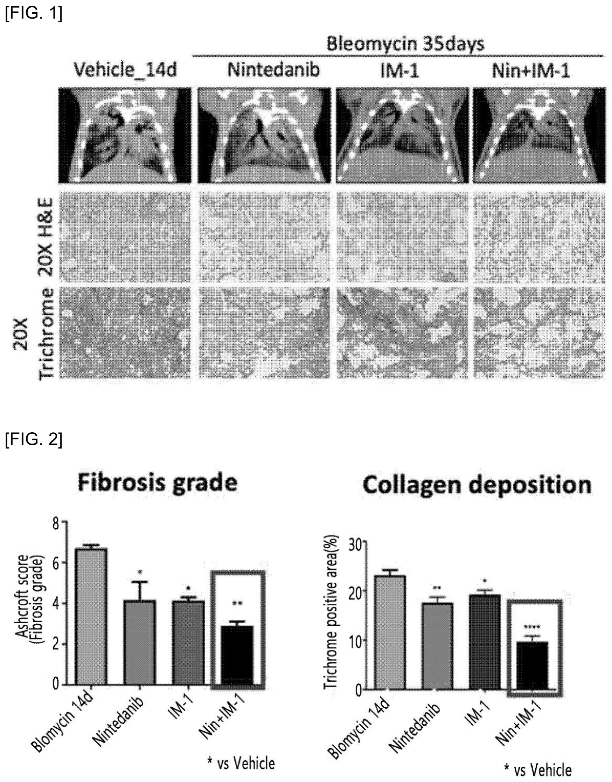

The results of Micro-CT imaging performed 4 weeks after bleomycin administration are shown in FIG. 1. Additionally, to analyze the degree of inflammatory response and the degree of fibrosis, H&E staining and Masson's Trichrome staining were performed. The fibrosis grade and collagen deposition are shown in FIG. 2.

As a result, it was confirmed that, in the untreated group, progression of fibrosis was observed two weeks after bleomycin administration, whereas in each of the group treated with nintedanib and the group treated with IM-1, a reduction in the degree of fibrosis was confirmed. However, the greatest effect was observed in the group treated with the combination of the two drugs.

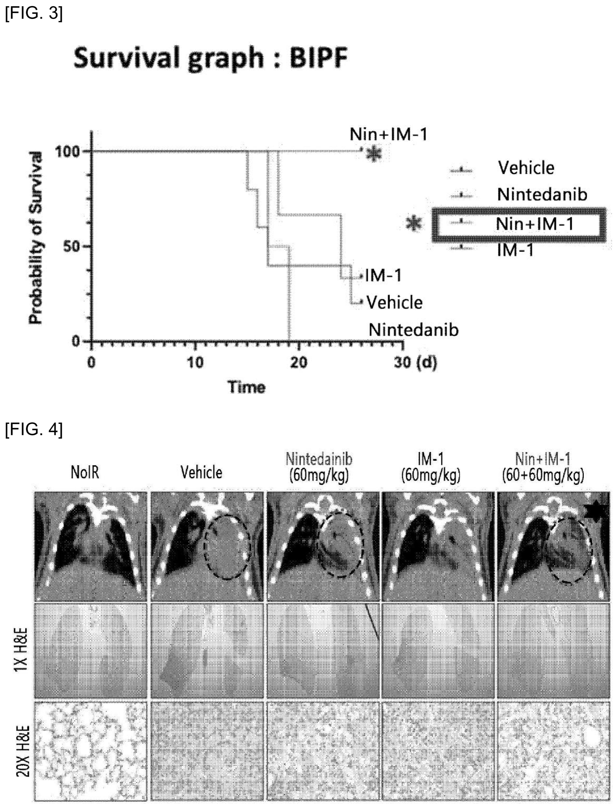

In addition, the mouse survival rate was compared in FIG. 3.

As a result of examining the survival rate for each treatment group, it was found that, compared to the untreated group, the survival rate increased in the group treated with the combination of nintedanib and IM-1. The survival rate of the single administration groups decreased after four weeks, whereas the group treated with the combination showed a survival rate of 100%.

From the above, it was confirmed that pulmonary fibrosis can be inhibited by the combination administration of the present invention.

### Example 2. Confirmation of inhibitory effect on radiation-induced pulmonary fibrosis

Seven-week-old male C57BL/6 mice were divided into groups treated with IM-1, nintedanib, and the combination of the two drugs (IM-1 + nintedanib). One hour before stereotactic body radiation therapy (SBRT; 90 Gy, 4 mm collimator), the drugs were orally administered to the mice. The degree of pulmonary fibrosis and the inhibitory effect on radiation-induced lung injury were observed two weeks after radiation exposure.

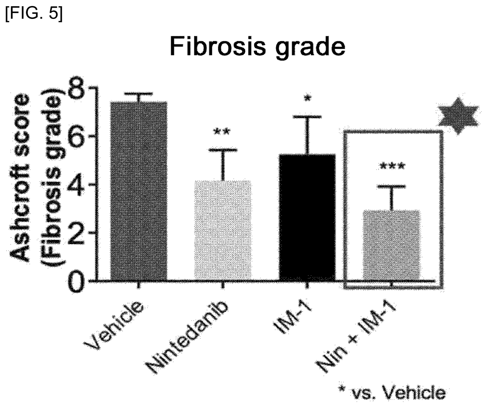

The IM-1 and nintedanib groups were each administered the respective drug daily at a dose of 60 mg/kg, and the combination group was administered both drugs daily at 60 mg/kg each. The results of Micro-CT imaging performed 2 weeks after radiation exposure are shown in FIG. 4. Additionally, to analyze the degree of inflammatory response and the degree of fibrosis, H&E staining and Masson's Trichrome staining were performed. The fibrosis grade and collagen deposition are shown in FIG. 5.

As a result of Micro-CT imaging two weeks after radiation exposure, it was confirmed that, in the untreated group, progression of fibrosis was observed two weeks after radiation exposure, whereas in each of the group treated with nintedanib and the group treated with IM-1, a reduction in the degree of fibrosis was confirmed compared to the untreated group. However, the greatest alleviation effect was observed in the group treated with the combination of the two drugs.

From the above description, those skilled in the art to which the present invention pertains would understand that the present invention can be implemented in other specific forms without changing its technical concept or essential features. Therefore, the embodiments described above should be construed as being exemplified and not limiting the present disclosure. The scope of the present invention should be interpreted to include all modifications or modified forms derived from the meaning and scope of the claims as set forth below, as well as equivalent concepts, rather than from the detailed description above.

## Claims

1. A pharmaceutical composition for preventing or treating pulmonary fibrosis, the pharmaceutical composition comprising a pharmaceutically effective amount of pexidartinib or a pharmaceutically acceptable salt or solvate thereof:
wherein the pharmaceutical composition is co-administered with nintedanib or a pharmaceutically acceptable salt or solvate thereof.

2. The pharmaceutical composition of claim 1, wherein the pexidartinib is a compound represented by following Formula 1:

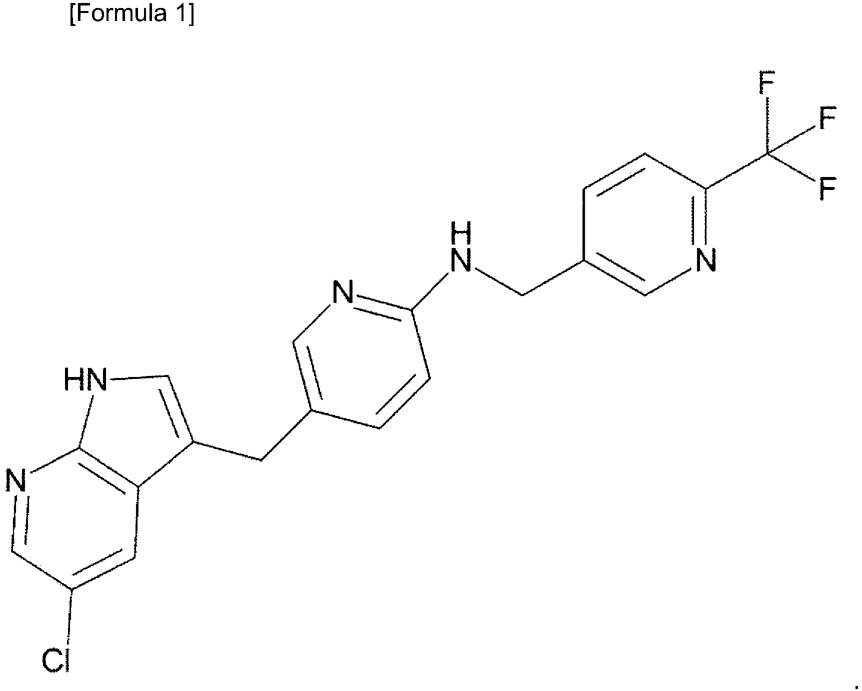

3. The pharmaceutical composition of claim 1, wherein the nintedanib is a compound represented by following Formula 2:

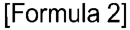

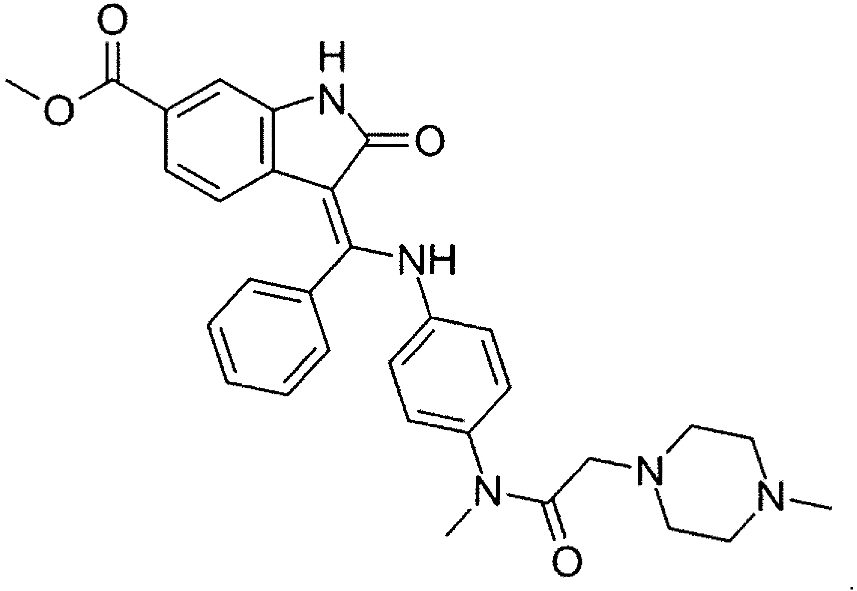

4. The pharmaceutical composition of claim 1, wherein the pharmaceutical composition further comprises a pharmaceutically acceptable carrier, excipient, or diluent.

5. The pharmaceutical composition of claim 1, wherein the pexidartinib or pharmaceutically acceptable salt or solvate thereof is administered with nintedanib or a pharmaceutically acceptable salt or solvate thereof concurrently, sequentially or in reverse order.
